(19) Europäisches Patentamt
European Patent Office
Office européen des brevets

(11) **EP 4 039 176 A1**

(12) **EUROPEAN PATENT APPLICATION**

(43) Date of publication:
**10.08.2022 Bulletin 2022/32**

(21) Application number: **21305147.7**

(22) Date of filing: **04.02.2021**

(51) International Patent Classification (IPC):
**A61B 5/00** (2006.01)   **A61B 5/16** (2006.01)
**A61B 5/375** (2021.01)   **A61B 5/08** (2006.01)
**A61B 5/11** (2006.01)

(52) Cooperative Patent Classification (CPC):
**A61B 5/0006; A61B 5/0008; A61B 5/16;**
**A61B 5/375; A61B 5/7275;** A61B 5/08; A61B 5/11;
A61B 5/163; A61B 5/389; A61B 5/486

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Open Mind Innovation SAS**
**27150 Hébécourt (FR)**

(72) Inventors:
• **Chatel-Goldman, Jonas**
**34570 Pignan (FR)**
• **Bertrand-Lalo, Raphaëlle**
**75017 Paris (FR)**

(74) Representative: **Cabinet Camus Lebkiri**
**25 rue de Maubeuge**
**75009 Paris (FR)**

(54) **GROUP BIOFEEDBACK METHOD AND ASSOCIATED SYSTEM**

(57) The invention relates to a method for providing biofeedback to a group of users, the method being carried out a plurality of times and comprising at least the following steps:
- Acquiring at least one biological signal using at least one biological sensor for each user of the group of users,
- Receiving, by a processor, the at least one biological signal of each user of the group of users,
- Computing, by the processor, a regularized interaction matrix of a dataset comprising at least part of the received biological signals,
- Computing, by the processor, a Riemannian distance between the regularized interaction matrix and a pre-computed regularized interaction matrix under no interaction,
- Providing biofeedback to the group of users using sensory means, the biofeedback being based on the computed Riemannian distance.

FIG. 1

**Description**

**TECHNICAL FIELD**

The technical field of the invention is the field of biofeedback methods.

The present document concerns a group biofeedback method which comprises the computation of a metric of interaction between users of the group, the metric being based on multivariate time series acquired by sensors.

**STATE OF THE ART**

Interaction measures are of paramount importance to monitoring the functioning of complex systems, both within the same system and between several of such systems. In practical applications implementing several systems, each system may issue several time-series of data. To measure the interaction of the different time-series, many bivariate interaction measures exist such as those reviewed in Congedo M (2018) "Non-Parametric Synchronization Measures used in EEG and MEG", GIPSA-lab, CNRS, University Grenoble-Alpes, Grenoble-INP.

Popular bivariate interaction measures include the Pearson's and Spearman's correlation coefficient, the coherence (sometimes named coherency), the mutual information rate, the phase-locking value. All those are intimately related. For instance, coherence is analogous to the Pearson's correlation coefficient in the frequency or time-frequency domain, whereas the phase-locking value is a non-linear version of it. Spearman's correlation coefficient is equivalent to the Pearson's correlation coefficient on ranked data. Pearson's correlation is a bivariate measure of the linear correlation between two variables, defined as the ratio between the covariance of the two variables and the square root of the product of their variance. It is comprised between -1 (perfect anti-correlation) and 1 (perfect correlation), passing through 0 (complete absence of correlation). Bivariate measures concern only the interaction of two time-series, thus they fail in providing a modular measure that readily scale to any number of time-series.

Extended interaction measures that do scale to multiple time-series exist, however in general they require many data points for their estimation, failing to provide a timely measure in online monitoring (that is, data processing under pseudo real-time constraint). Furthermore, in general they fail to provide an accurate measure in the presence of outliers, a problem that is exacerbated in online monitoring due to the difficulty of identifying outliers from the stream of incoming data.

Complex systems can be observed through various types of instruments, experimental setups, and sources simultaneously, providing a multi-faceted view of the system under scrutiny. Such a related set of measurements can be referred to as dataset. Extraction of multivariate interpretable features from a single dataset or from multiple related datasets can be used for classification, prediction, detection, and change analysis, among other tasks. There is increasing evidence from various fields (e.g., biomedical studies, environmental remote sensing, or cosmology, see for example Lahat, D., Adali, T., & Jutten, C. (2015) "Multimodal data fusion: an overview of methods, challenges, and prospects." Proceedings of the IEEE, 103(9), 1449-1477) that data integration across multiple datasets or subsystems allows to improve performance in such tasks by exploiting the joint information that exists among datasets, thereby revealing important relationships that cannot be detected by using a single modality. However, optimal use of multimodal data via data integration or fusion is still not a universally accepted way to study complex systems, due to the limited availability of multimodal datasets as well as due to the difficulty in combining data from different nature, see for example Calhoun, V. D., & Sui, J. (2016) "Multimodal fusion of brain imaging data: a key to finding the missing link (s) in complex mental illness." Biological psychiatry: cognitive neuroscience and neuroimaging, 1(3), 230-244.

Complex systems are inherently multiscale phenomenon, comprising a range of physical processes, ranging in length and time scales with multiple levels of description. For example, in biofeedback systems, that is in systems which provide auditory or visual feedback representative of measured physiological functions for a user to control said physiological functions, the measures of the physiological functions result in the creation of large datasets. When trying to provide biofeedback for a group of users, the dataset is even larger. In group biofeedback, there is a need for the monitoring of interaction between users. This calls for methodological approaches that can capture interaction in systems across different time scales and organizational levels. This multiscale challenge can be partially addressed by applying mathematical formalisms such as coordination dynamics or critical dynamics to the study of a complex system. However, the aforementioned approaches require extensive prior modelling, or they only explain a small portion in the revealed dynamics, which prevents from fully grasping the complex interplay of multiple system parts. In addition, computational approaches that can be tuned to selectively monitor interaction at a chosen level of description are sparse or non-existent.

To address the drawbacks mentioned before, there is a need for a solution able to measure interaction in groups of users and to provide this interaction information as biofeedback to said group of users.

## SUMMARY OF THE INVENTION

The present invention solves the above-mentioned problems by providing a solution able to provide proper biofeedback to a group of users.

According to a first aspect of the invention, this is satisfied with a method for providing biofeedback to a group of users, the method being carried out a plurality of times and comprising at least the following steps:

- Acquiring at least one biological signal using at least one biological sensor for each user of the group of users,

- Receiving, by a processor, the at least one biological signal of each user of the group of users,

- Computing, by the processor, a regularized interaction matrix of a dataset comprising at least part of the received biological signals,

- Computing, by the processor, a Riemannian distance between the regularized interaction matrix and a pre-computed regularized interaction matrix under no interaction,

- Providing biofeedback to the group of users using sensory means, the biofeedback being based on the computed Riemannian distance.

Thanks to the invention, it is possible to provide proper biofeedback to several users simultaneously through an interaction measure that provides enhanced metrics estimation, and that enables for multi-modal integration and multi-scale integration in a modular and flexible way. More specifically, the invention:

- i. requires a low number of data-points for its estimation,

- ii. is not affected by the autocorrelation of time-series,

- iii. is robust to outliers,

- iv. can be applied to heterogeneous datasets obtained from multiple sensor modalities.

In order to define an interaction measure that requires a low number of data-points for its estimation and that is robust to outliers, the present invention is based upon advances on the Riemannian geometry of positive-definite matrices. In order not to be affected by the autocorrelation of time-series, a regularization method in conjunction with Riemannian geometry is adopted.

Matching characteristics iv. is performed by computing pairwise interaction measure from raw data obtained directly from sensors and/or from time-varying features extracted from raw data after a pre-processing stage.

The invention permits to carry out real-time or near real-time analysis of bio-signals from multiple users and to provide group biofeedback to said users based on an interaction metric resulting from the real-time or near real-time analysis.

The method according to the invention may also have one or more of the following characteristics, considered individually or according to any technically possible combinations thereof:

- the computed interaction matrix is a positive-definite matrix such as a cross-covariance matrix, Pearson's correlation matrix, a Spearman's correlation matrix or a Kendall's correlation matrix .

- the method further comprises, after the step of computing the regularized interaction matrix, a step of computing, by the processor, a smoothed regularized interaction matrix from the regularized interaction matrix and in that, at the step of computing a Riemannian distance, the computed Riemannian distance between the regularized smoothed interaction matrix and a pre-computed regularized smoothed interaction matrix under no interaction.

- the step of computing a smoothed regularized interaction matrix comprises applying a smoothing operation in the time domain using a Riemannian process.

- the at least one biological signal is a signal representative of at least one activity chosen from the following list: electroencephalography, electrocardiography, electrodermal activity, respiratory activity, eye-tracking, electromyographic activity, electro-oculographic activity, body motion.

- the method comprises a step of issuing a report comprising at least the computed Riemannian distance at each run of the method.

Another aspect of the invention relates to a system for providing biofeedback to a group of users, the system comprising:

- At least one biological sensor for each user of the group of user, each sensor being configured to carry out the step of acquiring at least one biological signal of the method according to the invention,

- At least one processor configured to carry out the step of receiving the biological signal and the computing steps of the method according to the invention,

- Sensory means configured to carry out the step of providing biofeedback according to the computed Riemannian distance of the method according to the invention.

The system according to the invention may also have one or more of the following characteristics, considered individually or according to any technically possible combinations thereof:

- the sensory means are chosen from the following list: a sound producing device, a display device, a vibro-tactile device.

- the biological sensors are chosen from the following list : an electroencephalograph, an electrocardiograph, an electrodermal activity sensor, a respiratory activity sensor, an eye-tracker, an electromyograph, electrooculograph, a body motion sensor

Another aspect of the invention relates to a computer program product comprising instructions which, when the program is executed by a computer, causes the computer to carry out the method according to the invention.
Another aspect of the invention relates to a computer-readable medium comprising instructions which, when executed by a computer, cause the computer to carry out the method according to the invention.
The invention finds a particular interest to assess and improve the interaction among users, for example co-workers, in a group biofeedback session.

## BRIEF DESCRIPTION OF THE FIGURES

Other characteristics and advantages of the invention will become clear from the description that is given thereof below, by way of indication and in no way limiting, with reference to the appended figures, among which:

- Figure 1 is a schematic representation of a system configured to carry out the group biofeedback method according to the invention,

- Figure 2 is a schematic representation of a group biofeedback method according to the invention,

- Figure 3 shows the resulting measures of an implementation of the invention.

## DETAILED DESCRIPTION

For greater clarity, identical or similar elements are marked by identical reference signs in all of the figures.
The figures are provided for information purposes only and are in no way limiting the invention to their disclosure.

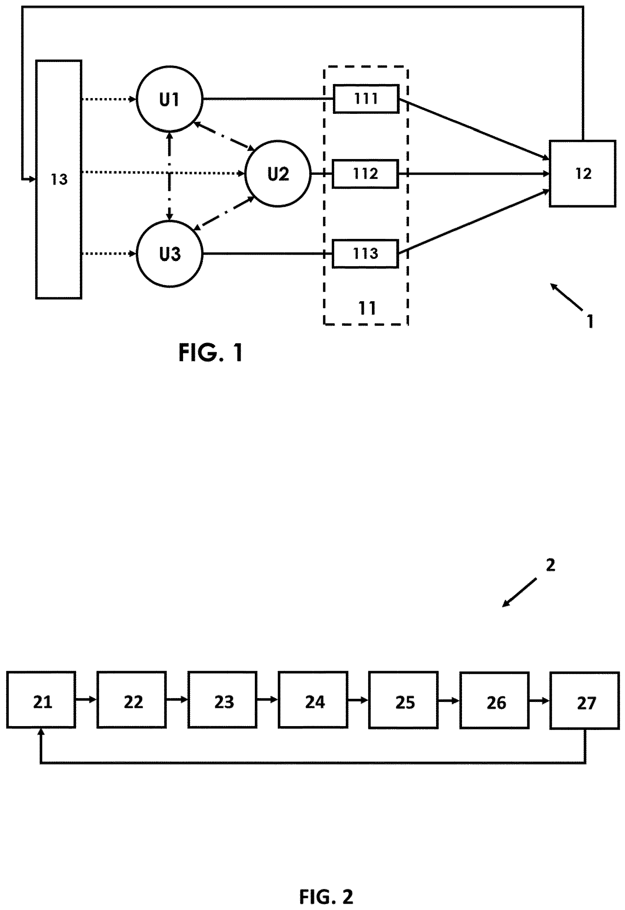

Figure 1 presents the system configured to carry out the group biofeedback method of the invention.
The system 1 represented at Figure 1 comprises a plurality of biological sensors 11, a processor 12 and sensory means 13.
The group biofeedback system 1 is used by users U1 to U3. The users U1 to U3 are interacting, for example through a common task, or by simply talking, touching, or performing any other action where they interact. Such interaction can occur in the same room or real environment or in a virtual environment. For each user U1 to U3, at least one sensor is configured to acquire at least one biological signal of said user. For example, as represented at Figure 1, a biological signal of user U1 is acquired by a sensor 111, a biological signal of user U2 is acquired by a sensor 112 and a biological signal of user U3 is acquired by a sensor 113. Preferably, a plurality of sensors acquire a plurality of biological signals for each user, enabling a deeper analysis based on more data for each user.

It is understood by "biological signal" of a user a signal representative of at least one biological activity of a user. For example, a biological signal can be a signal representative of an electroencephalography, an electrocardiography, an electrodermal activity, a respiratory activity, an eye-tracking, an electromyographic activity, an electro-oculographic activity, a body motion. A signal is representative of an activity when it represents the activity. For example, for an electroencephalography, the resulting signals acquired by the different electrodes of the encephalograph together are the biological signals representative of an electroencephalography. Biological signals are preferably electrical signals, but can take any other form of biological signals. The biological signals are time-series, that is a series of data points indexed in time order. More specifically, in the invention, a time series is a sequence of data point acquired at successive samples that are approximately equally spaced in time. A sample is a recording at a time instant, used referring to both the recording of one variable or of many variables. The ensemble of biological signals for a single user or for different users form a multivariate time-series. A multivariate time-series is a time-series acquired on several variables, either several recording points from the same system and/or several recording points from each of several systems, where each sample of all variables are acquired at the same time instant, implying that the time-series are acquired simultaneously and synchronously in time.

The plurality of biological sensors 11 configured to acquire the biological signals are attached to, placed on, or located near a user. The biological sensors 11 can take any form, they can for example be embedded in a helmet, attached using adhesives or dry contact. The invention is not limited to any type or form of biological sensor 11. The biological sensors 11 can be taken from the following list, alone or in combination: an electroencephalograph, an electrocardiograph, an electrodermal activity sensor, a respiratory activity sensor, an eye-tracker, an electromyograph, electrooculograph, a body motion sensor.

Two users preferably carry the same type of sensors 11, but the invention is not limited to this embodiment and is able to process different types of data from different users carrying different types of forms of biological sensors 11.

Another component of the group biofeedback system 10a is a processor 12. The processor 12, coupled with a memory (not represented), is configured to carry out the steps of the group biofeedback method of the invention. The memory stores instructions which, when executed by the processor 12, cause the processor 12 to carry out the steps of the method according to the invention. The processor 12 receives data from the biological sensors 11 via wired means, or wirelessly. This can be done through a network, requiring network interfaces on the processor 12 and on the sensors 11 sides. The data acquired by the sensors can all be sent to the processor 12 through the same network interface or through different network interfaces when each user carries a sensor system comprising its own network interface. The signals received by the processor 12 from the sensors 11 are concatenated to form an input multivariate signal.

The processor 12 is further connected to sensory means 13. This connection can be wired or wireless, through a network or locally. The processor 12 then instructs the sensory means 13 to provide a sensory feedback to users U1 to U3, depending on the result of the method according to the invention carried out by the processor 12. This instruction can be direct, with the processor being directly connected to the sensory means 13, or indirect, with the processor 12 sending a piece of information to another device, which then decides what to instruct the sensory means 13 so that they provide proper sensory feedback to the users U1 to U3.

The sensory means can be any device, system or component configured to provide a sensory feedback to users U1 to U3. The sensory feedback can be individual, being the same for each user U1 to U3, or can be a group feedback. For example, the sensory means can be visual, for example using a screen, which can be seen by all the users U1 to U3 at the same time. On the other hand, the sensory means 13 can be for example a Virtual Reality ("VR") headset, providing the same feedback to each user U1 to U3 independently, each user carrying a VR headset. Sensory means 13 can be taken from the following list, alone or in combination, without being limited to said list: a sound producing device such as speakers or headphones, a display device such as a screen or a VR headset, a vibro-tactile device, an electrical stimulation device.

Figure 2 is a schematic representation of a group biofeedback method according to the invention.

The group biofeedback method 2 represented at Figure 2 comprises seven steps. Some of the steps of the method can be omitted, this will be specified further in the description.

In a first step 21 of the group biofeedback method 2, the biological sensors 11 acquire at least one biological signal for each of the users U1 to U3. The biological signals are multivariate time-series. Let $x(t) \in \mathbb{R}^n$ be the continuously acquired multivariate time-series measurements. This is a real (or complex) vector holding n components at each time sample t. We denote by $X \in \mathbb{R}^{n \cdot T}$ a time window of the measurement comprising T samples. In the following the measurement x(t) is assumed real and continuously filtered in a frequency band-pass region of interest. This effectively makes the time-series having zero mean.

The group biofeedback method 2 then comprises a step 22 of receiving, by the processor 12, the at least one biological signal of each user of the group of users U1 to U3. This is done using the network interfaces previously mentioned and/or via any wired or wireless transmission mean.

At a step 23, the processor 12 computes a regularized interaction matrix of the dataset x(t) comprising the received biological signals. The dataset x(t) can comprise only part of the acquired biological signals, or can comprise other signals than only the received biological signals.

The computing of the regularized interaction matrix will now be described. Let $C = T^{-1}XX^T \in \mathbb{R}^{n \cdot n}$ be the sample covariance matrix of X, where X is a time window of the measurement comprising T samples as defined previously. If the time-series are uncorrelated then the covariance matrix is diagonal. A global measure of interaction is therefore given by a distance measure between C and its diagonal part, denoted D=diag(C). Riemannian geometry provides a natural way to measure such distance, denoted $\delta(C,D)$, which will be described at step 24.

For a better understanding of the context and of the rest of the description, the following definitions are needed:

- Riemannian geometry is the branch of differential geometry that studies Riemannian manifolds.

- A Riemannian manifold is a differentiable topological space that is locally homeomorphic to a Euclidean space, over which a tangent space can be defined at any point in the manifold. The definition of a scalar product for the vectors in the tangent space allows the definition of a Riemannian geometry for the manifold.

- The tangent space at a point G refers to the collection of all tangent vectors to all curves on the manifold passing through G.

- A geodesic is the shortest path relying two points P and Q on a manifold, analogous of straight lines in Euclidean spaces. The distance is the length of the geodesic relying two points P and Q on a manifold, while the mean is the mid-point G on the geodesic relying two points P and Q. For a given distance function the mean is the minimizer of $\delta^2(P, G) + \delta^2(Q, G)$.

- Using Frechet's variational approach it is possible to extend to positive-definite matrices the concept of mean of a set of scalars; as the midpoint G on the geodesic relying P and Q is the minimizer of $\delta^2(P, G) + \delta^2(Q, G)$, so the Frechet mean of k points $P_1$, $P_2$, ..., $P_k$ is the matrix F verifying : $\mathrm{argmin}_{(F)} \Sigma_{i=1}{}^{k}(\delta^2(P_i, F))$.

- An affine-invariant metric refers to a metric that possesses the affine-invariance property. if P and Q are two positive definite matrices and $\delta(P, Q)$ is the distance between P and Q that results from the metric, the metric is said affine-invariant if $\delta(P, Q) = \delta(BPB^H, BQB^H)$ for any non-singular matrix B.

- A positive definite matrix is a real or complex square matrix which eigenvalues are all real and strictly positive. If the matrix is real it is symmetric. If it is complex it is Hermitian. Elements $p_{ij}$ of symmetric matrix P are such that $p_{ij} = p_{ji}$, which implies that $P = P^T$, where superscript T denotes matrix transposition. Elements $p_{ij}$ of Hermitian matrix P are such that $p_{ij} = p_{ji}{}^*$, where superscript * denotes the complex conjugate of a complex number, implying $P = P^H$, where superscript H denotes complex-conjugate and transpose. In this invention we will use superscript T referring to the case of real matrices and superscript H only when referring to complex matrices. For the general case, that is, when not otherwise specified, superscript T will be used for simplicity. Both real and complex positive definite matrices may be equipped with an affine-invariant metric allowing the definition of a Riemannian manifold.

- For a positive-definite matrix P there is a unique symmetric (if P is real) or Hermitian (if P is complex) positive definite matrix $P^{0.5}$ such that $P = P^{0.5}P^{0.5}$. Such matrix is named the principal square root of P.

Given several time series, the Pearson's correlation matrix holds at the (i,j) entries the Pearson's correlation between time series i and time-series j. The matrix is symmetric and has 1s on the diagonal (since the Pearson's correlation of a series with itself is always 1). An interaction matrix of the invention is analogous to Pearson's correlation matrix, but using another bivariate interaction measure to fill the (i,j) entries. Examples of such measures are Spearman's and Kendall's correlation, the normalized mutual information, etc. For instance, Spearman's correlation is sensitive to any monotonic correlation, not just linear correlation, thus for time-series which dependence is monotonic, but not linear, it provides a more suitable interaction measure.

Therefore, if C is the covariance matrix of the data and D the diagonal matrix holding its diagonal elements, then $R = D^{-0.5}CD^{-0.5}$ is Pearson's correlation matrix of X, which is a suitable interaction matrix. Other interaction matrices may also be used, using correlations such as Spearman's or Kendall's correlation, thus giving the Spearman's correlation matrix or Kendall's correlation matrix.

In an optional step 24, a smoothing operation is performed on the obtained interaction matrix.

For continuous monitoring of the interaction, the invention considers sliding windows, that is, windows of the chosen duration T (in sample), continuously built starting at successive incoming samples t=1, 2,...,n.

For each window starting at time t yielding regularized interaction matrix S(t) the smoothing operation can be performed using the following formula:

$$S(t) \leftarrow S(t\text{-}1)^{\,0.5} \, (S(t\text{-}1)^{\,-0.5} \, S(t) \, S(t\text{-}1)^{\,-0.5})^{\alpha} \, S(t\text{-}1)^{\,0.5}$$

where $0<\alpha<1$ is a smoothing parameter.

This formula is a move on the geodesic in the manifold of positive definite matrices relying S(t-1) to S(t). It always exists and it is unique ; with $\alpha=0$ there is no learning, that is, S(t) = S(t-1) whereas with $\alpha=1$ there is no smoothing, that is, S(t) = S(t). With $\alpha=0.5$, $S_t$ is the mid-point on the geodesic. The optimal value of $\alpha$ is to be adjusted depending on the applications. The geodesic equation is the Riemannian equivalent of a moving average process in the Euclidean geometry : $(1-\alpha) \, S(t\text{-}1)+\alpha S(t)$.

At a step 25, the processor 12 carries out the computing of a Riemannian distance between the (smoothed or not) regularized interaction matrix and a pre-computed regularized interaction matrix under no interaction.

The invented interaction measures are specific instances of the distance function with form

$$\delta(\mathrm{P}, \mathrm{Q}) = \sqrt{\sum_n log^2 \left( \lambda_n \left( \mathrm{P}^{\frac{-1}{2}} \mathrm{Q} \mathrm{P}^{\frac{-1}{2}} \right) \right)} \qquad (1)$$

where $\lambda_n(.)$ is the $n^{th}$ eigenvalues of the argument, $P^{-0.5}$ is the inverse of the principal square root of P and $log^2$ is the square of the logarithm.

Distance (1) corresponds to the distance adopting the Fisher affine-invariant metric on the manifold of positive-definite matrices. While other distance measures may be employed, there are a number of reasons for adopting this metric that makes it particularly appealing in theory and in practice when working with real data:

- Besides verifying the affine-invariance property mentioned previously, this metric verifies also the invariance under inversion, that is, for any two positive-definite matrices P and Q, $\delta(P, Q) = \delta(P^{-1}, Q^{-1})$.

- This metric has been the object of intense studies in mathematics due to its appealing theoretical properties.

- This metric is the natural choice when the time-series follows a multivariate Gaussian process, which is a very common and well-understood theoretical framework.

The distance usually employed is the Euclidean distance $\varepsilon(P,Q) = \|P\text{-}Q\|$ where $\| \|$ indicates the Frobenius norm. This distance however does not possess either of these properties.

As explained before, the Pearson's correlation matrix R of X in terms of the covariance matrix C is $R = D^{-0.5}CD^{-0.5}$. Using this formula and the affine-invariant property of the Fisher metric we have $\delta(C,D) = \delta(R, I)$, where I is the identity matrix, i.e. the counterpart of the interaction matrix R under no interaction, used as a reference on the manifold of positive-definite matrices. This counterpart interaction matrix under no interaction can for example be pre-computed. Therefore, in this case the interaction measure is given equivalently by

$$\delta(\mathrm{R}, \mathrm{I}) = \sqrt{\sum_n log^2 \left( \lambda_n(\mathrm{R}) \right)} \quad (1)$$

where $\lambda_1(R),..., \lambda_n(R)$ are the n eigenvalues of R.

When n is large and the time series are correlated, depending also on the number of available samples T, matrix R may be ill-conditioned, meaning that the smallest eigenvalues are close to zero. This may cause numerical problems in the computation of the $\delta$ distance and may also leads to biased measures. For this reason, this invention considers instead a regularized version of the interaction measures with form (1), given by

$$\delta(S, I) = \sqrt{\sum_n \log^2(\lambda_n(S))} \quad (2)$$

Where S = D'$^{-0.5}$C' D'$^{-0.5}$, C' = C + γI, D' = D + γI,
with γ the regularization parameter.

As explained before, for continuous monitoring of the interaction measure defined, sliding windows of the incoming time-series data are considered, the interaction matrix of the incoming data windows are estimated and smoothed using past estimations.

An advantage of the framework defined by equation (1) is that it easily adapts to several case scenarios, as will be shown, including situations where there are multivariate measurements from several dependent and independent systems to be observed and when the system(s) is to be monitored at the sub-system level or at several band-pass frequency regions simultaneously.

It can be noted that in the special case n=2 the defined interaction measure is a function of the bivariate measure used to build the interaction matrix. For instance, with the Pearson's correlation matrix as the interaction matrix, the global interaction measure is a (non-linear) function of the Pearson's correlation coefficient.

At a step 26, biofeedback is provided to the group of users U1-U3 using the sensory means 13, the biofeedback being based on the computed Riemannian distance. The sensory means 13 have been described previously. At step 26, the sensory means provide a feedback, for example a visual, audio or tactile feedback, the feedback depending on the computed Riemannian distance. This means that the feedback can be louder, stronger, or can change if the Riemannian distance verifies a certain criterion, or on the opposite can be quieter, weaker or can change in another way if the Riemannian distance does not verify a certain criterion. This feedback affects the users U1 to U3, which in turn causes a biological change, which reflects on the following computed Riemannian distance(s). This permits a better interaction between the users U1 to U3. Feedback systems operates in pseudo real-time with minimal delay to allow using online monitoring of global interaction for purpose of learning or remediation.

At an optional step 27, a report comprising at least the computed Riemannian distance is issued at each run of the method. The report can be issued by the processor 12, or by a non-represented device or component that receives the computed Riemannian distance by the processor 12.

The invention can further have different embodiments:

In one embodiment, the global regularized interaction measure defined can be split in several local measures. To do so, a desired number of subsets of the *n* time series is considered and the interaction measure is computed for all of them. The subsets can be taken in any permutation as the distance is invariant to permutation of the time-series. These local measures may be further aggregated or combined, depending on the application.

In one embodiment, multiple frequency band-pass regions are considered for the time-series and the regularized interaction matrix S is obtained for all of them. Frequency-specific measures may be further aggregated or combined, depending on the application.

In one embodiment, the time series x(t) records several independent phenomena of the same system, each one allowing one or more recordings. In this case, $x(t) \in \mathbb{R}^n$, n=n$_1$+,...,+n$_p$, where p is the number of independent phenomena to be monitored and n$_1$,...,n$_p$ is the number of time-series recoded on each phenomenon. Since the p systems are independent from each other, matrix S is this embodiment has a block-diagonal structure, such as :

$$S^w = \begin{pmatrix} S_1 & \cdots & 0 \\ \vdots & \ddots & \vdots \\ 0 & \cdots & S_p \end{pmatrix}$$

The eigenvalues of a block diagonal matrix are the eigenvalues of the blocks, thus the global interaction measure in this case takes the following form:

$$\delta(S^w, I) = \sqrt{\sum_p \sum_n \log^2\left(\lambda_n(S_p)\right)} \quad (3)$$

In one embodiment, the time series x(t) records several systems and the dependencies among these systems is of interest, besides, or instead of, the dependency of the time-series within each system. In this case also $x(t) \in \mathbb{R}^n$, $n = n_1 + , \ldots , + n_p$, where p is the number of possibly dependent phenomena to be monitored and $n_1, \ldots, n_p$ is the number of recordings for each phenomenon. However, in this embodiment matrix S has to be considered in its full partitioned form, that is :

$$S^t = \begin{pmatrix} S_1 & \cdots & S_{1p} \\ \vdots & \ddots & \vdots \\ S_{p1} & \cdots & S_p \end{pmatrix},$$

where the diagonal blocks $S_i$ hold the dependency within-system i and off-diagonal blocks $S_{ij}$ hold the dependency between system i and system j. Using the interaction measure of Eq. (2) on $S^t$ thus defined describes both the dependency within the systems and between the systems. It is referred to the ensuing invented measure of interaction as the total interaction measure:

$$\delta(S^t, I) = \sqrt{\sum_n log^2(\lambda_n(S^t))} \qquad (4)$$

In one embodiment the within- and between-system dependencies are separated. The within-system dependency is defined as per equation (3). For the between-system dependency, the regularized global interaction matrix of the data $S^t$ and the block-diagonal matrix holding the p regularized covariance matrices of each system $S^w$ are considered. Their distance defines the between-system dependency, such as:

$$\delta(S^w, S^t) \ (5)$$

In a biofeedback implementation example, the invention was carried out to obtain:

- High reactivity

- Robustness to noise

- Ability to online pursuit the total, between-system and within-system interaction of a complex system formed by two agents providing synchronized multivariate time-series measurement.

With the chosen parameters:

- $\gamma = 0.1$,

- $\alpha = 0.75$,

- T = 1s.

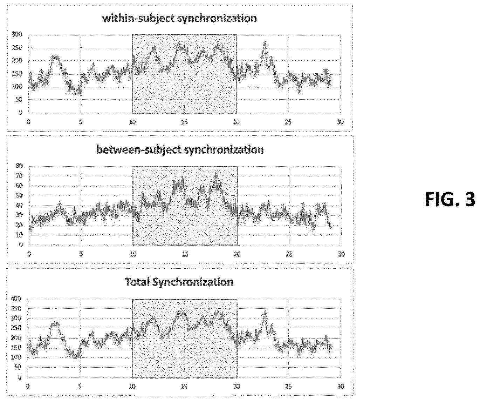

The data for this example comes from an experiment where the electroencephalography (EEG) was recorded synchronously on two healthy adults. Ten electrodes were used for EEG recording on each subject, placed at standard EEG scalp positions P7, P3, PZ, P4, P8, PO3, PO4, O1, 0z and 02, which cover evenly the occipital cortex of the brain. The experiment follows an ABA design, where condition A (10s each) consists of a resting state with the eyes open. In condition B (10s) a flashing light flickering at 13Hz was placed in front of both subjects. The flickering light is known to engender an interaction of areas of the visual cortex at the frequency of the flickering. This in turn causes within-subject interaction of the EEG recording, but also between-subject interaction of the EEG recording because the flickering is perceived and processed synchronously by the two subjects. EEG data was recorded at 128 samples per seconds and filtered in the frequency band-pass 12-14Hz. The result of applying the presently invented total interaction measure (3), within-system (subjects in this example) interaction measure (4) and between-system interaction measure (5) is shown in Figure 3, where the x-axis is time in seconds and the y-axis is the interaction measure. On the average, all interaction measures are higher in condition B (indicated by the transparent box) as compared to the two control conditions A. Also, the between-subject interaction measure is rather stable in conditions A as compared to condition B, while the within-subject interaction measure fluctuates in all conditions, indicating that during conditions A, when between-subject interaction is not induced by the flickering light, the dynamic range of between-subject dependency is lower as compared to the within-subject dependency, which is an intrinsic property of the brain in any conditions.

**Claims**

1. Method for providing biofeedback to a group of users, the method being carried out a plurality of times and comprising at least the following steps:

   - Acquiring at least one biological signal using at least one biological sensor for each user of the group of users,
   - Receiving, by a processor, the at least one biological signal of each user of the group of users,
   - Computing, by the processor, a regularized interaction matrix of a dataset comprising at least part of the received biological signals,
   - Computing, by the processor, a Riemannian distance between the regularized interaction matrix and a pre-computed regularized interaction matrix under no interaction,
   - Providing biofeedback to the group of users using sensory means, the biofeedback being based on the computed Riemannian distance.

2. Method according to the preceding claim **characterized in that** the computed interaction matrix is a positive-definite matrix.

3. Method according to the preceding claim **characterized in that** the computed interaction matrix is a cross-covariance matrix, a Pearson's correlation matrix, a Spearman's correlation matrix or a Kendall's correlation matrix.

4. Method according to any of the preceding claims **characterized in that** it further comprises, after the step of computing the regularized interaction matrix, a step of computing, by the processor, a smoothed regularized interaction matrix from the regularized interaction matrix and **in that**, at the step of computing a Riemannian distance, the computed Riemannian distance between the regularized smoothed interaction matrix and a pre-computed regularized smoothed interaction matrix under no interaction.

5. Method according to any of the preceding claims **characterized in that** the step of computing a smoothed regularized interaction matrix comprises applying a smoothing operation in the time domain using a Riemannian process.

6. Method according to any of the preceding claims **characterized in that** the at least one biological signal is a signal representative of at least one activity chosen from the following list: electroencephalography, electrocardiography, electrodermal activity, respiratory activity, eye-tracking, electromyographic activity, electro-oculographic activity, body motion.

7. Method according to any of the preceding claims **characterized in that** it comprises a step of issuing a report comprising at least the computed Riemannian distance at each run of the method.

8. System for providing biofeedback to a group of users, the system comprising:

   - At least one biological sensor for each user of the group of user, each sensor being configured to carry out the step of acquiring at least one biological signal of the method according to any of the preceding claims,
   - At least one processor configured to carry out the step of receiving the biological signal and the computing steps of the method according to any of the preceding claims,
   - Sensory means configured to carry out the step of providing biofeedback according to the computed Riemannian distance of the method according to any of the preceding claims.

9. System according to claim 8, **characterized in that** the sensory means are chosen from the following list: a sound producing device, a display device, a vibro-tactile device, an electrical stimulation device.

10. System according to any of claims 8 or 9, **characterized in that** the biological sensors are chosen from the following list : an electroencephalograph, an electrocardiograph, an electrodermal activity sensor, a respiratory activity sensor, an eye-tracker, an electromyograph, electro-oculograph, a body motion sensor.

11. Computer program product comprising instructions which, when the program is executed by a computer, causes the computer to carry out the method according to any one of claims 1 to 7.

12. Computer-readable medium comprising instructions which, when executed by a computer, cause the computer to carry out the method according to any one of claims 1 to 7.

FIG. 1

FIG. 2

within-subject synchronization

between-subject synchronization

**Total Synchronization**

**FIG. 3**

Europäisches
Patentamt

European
Patent Office

Office européen
des brevets

# EUROPEAN SEARCH REPORT

**Application Number**

EP 21 30 5147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MCCRATY ROLLIN: "New Frontiers in Heart Rate Variability and Social Coherence Research: Techniques, Technologies, and Implications for Improving Group Dynamics and Outcomes", FRONTIERS IN PUBLIC HEALTH, vol. 5, 12 October 2017 (2017-10-12), XP055826998, ISSN: 2296-2565, DOI: 10.3389/fpubh.2017.00267 Retrieved from the Internet: URL:http://dx.doi.org/10.3389/fpubh.2017.00267> * pages 4-5, 7-8 * | 1-12 | INV. A61B5/00 A61B5/16 A61B5/375 ADD. A61B5/08 A61B5/11 |
| X | KYUNG YUN CHOI ET AL: "reSpire", CREATIVITY AND COGNITION, ACM, 2 PENN PLAZA, SUITE 701NEW YORKNY10121-0701USA, 13 June 2019 (2019-06-13), pages 449-454, XP058439079, DOI: 10.1145/3325480.3329176 ISBN: 978-1-4503-5917-7 * pages 452-453; figures 3,8 * | 1-12 | |
| A | KR 2018 0099984 A (UNIV INDUSTRY FOUNDATION YONSEI UNIV WONJU CAMPUS [KR]) 6 September 2018 (2018-09-06) * paragraphs [0002] - [0035], [0051], [0079] - [0082] * | 1,6,8, 10-12 | TECHNICAL FIELDS SEARCHED (IPC) A61B |
| A | WO 2014/059234 A1 (UNIV CITY NEW YORK RES FOUND [US]) 17 April 2014 (2014-04-17) * paragraphs [0014], [0031], [0105] - [0112] * | 1,8,11, 12 | |

The present search report has been drawn up for all claims

| Place of search | Date of completion of the search | Examiner |
|---|---|---|
| Berlin | 22 July 2021 | Dydenko, Igor |

## ANNEX TO THE EUROPEAN SEARCH REPORT
## ON EUROPEAN PATENT APPLICATION NO.

EP 21 30 5147

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2021

| Patent document cited in search report | Publication date | Patent family member(s) | | Publication date |
|---|---|---|---|---|
| KR 20180099984 A | 06-09-2018 | NONE | | |
| WO 2014059234 A1 | 17-04-2014 | CA | 2886597 A1 | 17-04-2014 |
| | | EP | 2906114 A1 | 19-08-2015 |
| | | US | 2015248615 A1 | 03-09-2015 |
| | | WO | 2014059234 A1 | 17-04-2014 |

EPO FORM P0459

For more details about this annex : see Official Journal of the European Patent Office, No. 12/82

**REFERENCES CITED IN THE DESCRIPTION**

**Non-patent literature cited in the description**

- Non-Parametric Synchronization Measures used in EEG and MEG. **CONGEDO M.** GIPSA-lab, CNRS. University Grenoble-Alpes, Grenoble-INP, 2018
- **LAHAT, D. ; ADALI, T. ; JUTTEN, C.** Multimodal data fusion: an overview of methods, challenges, and prospects. *Proceedings of the IEEE,* 2015, vol. 103 (9), 1449-1477
- **CALHOUN, V. D. ; SUI, J.** Multimodal fusion of brain imaging data: a key to finding the missing link (s) in complex mental illness. *Biological psychiatry: cognitive neuroscience and neuroimaging,* 2016, vol. 1 (3), 230-244